# EUROPEAN PATENT APPLICATION

(11) **EP 4 549 923 A1**
(43) Date of publication of application: **07.05.2025**
(21) Application number: 24210000.6
(22) Date of filing: 30.10.2024
(51) Int. Cl.: G01N 27/327

(54) **NANOTECHNOLOGY-ENABLED PRINTABLE BIOSENSING DEVICE FOR DISEASES DETECTION AND METHOD OF PRODUCING THEREOF**

(30) Priority: 30.10.2023 GR 20230100901
(71) Applicant: BL Nanobiomed Private Company, 57001 Thermi Thessalonikis (GR)
(72) Inventor: LOGOTHETIDIS, Stergios, 54642 Thessaloniki (GR); KARAGKIOZAKI, Varvara, 54646 Thessaloniki (GR); TSIMENIDIS, Konstantinos, 55131 Kalamaria, Thessaloniki (GR); ORFANOS, Alexandros, 56121 Ampelokipoi, Thessaloniki (GR)
(74) Representative: Petsis, Christos

(57) **Abstract**

A biosensing device for diseases detection including for diagnoses, monitoring and screening thereof at nanoscale, comprising a nano-technology enabled biosensing means, which is biofunctionalized for detection of a selected set of biomarkers and for quantitative measurements thereof, and an electronic device (110) with internet connectivity for data transmission, which is remarkable in that it comprises Flexible Substrate (101), Contacts-Electrodes (102), a Reference Electrode (103), Working & Counter Electrodes (104), Dielectric means (105) and a Biofunctionalization means (106), arranged as a set of successive layers, wherein said electrode means (102) are arranged onto said flexible substrate (101), esp. as a stack structure consisting of a superposition of said layers, further comprising insulation means consisting of an insulation layer provided between said electrodes means. Said biofunctionalization means (106) detects said biomarkers and monitors same.

## Description

### Field of the invention

The present invention relates to a fully printable, flexible, portable biosensor for diagnosis, monitoring and screening of diseases or other conditions with high selectivity and accuracy, even at nanoscale, with rapid response time, and to the production thereof.

### Background of the invention

In clinical diagnostics, rapid, easy, accurate, and low-cost detection of biomarkers are important target analytes. For instance, recent methods employed in immunoassays, such as enzyme-linked immunosorbent assay (ELISA) for the biomarker detection in body fluids such as urine, blood, and serum, serve valid and sensitive results. However, the long and time consuming procedures, the need for large amounts of samples and reagents, in line with the necessity for skilled personnel, prevent these devices from being used in resource-limited settings. Therefore, there is a critical demand for simple and affordable detection systems that are user-friendly with the capability to simply detect biomarkers and analyze them in resource-poor environments [1, 2].

Some paper-based biosensor applications such as diseases diagnosis, monitoring health conditions, pathogens detection, are one of the alternative approaches for clinical environment. Paper-based electrochemical analytical devices (ePADs) have gained significant interest as promising analytical units in recent years. Especially, paper-based electrochemical biosensors are attractive analytical devices since they can promote diagnose several diseases and potentially allow decentralized analysis. Electrochemical biosensors are versatile, as the measured signal can be improved by using mainly molecular technologies and nanomaterials to attach biomolecules, resulting in an increase of their sensitivity and selectivity.

Despite rigorous works on such biosensors, responses obtained are mainly qualitative or semi-quantitative results, unfortunately, that still need further improvements.

Biosensing mechanisms are also a key factor in improving biosensor performances by signal amplification strategies, which are determining parameters for the biosensor quality in clinical practice. Unfortunately, in recent conditions, paper-based devices are still left behind owing to several drawbacks. Signals to noise ratio plays an important role in biosensing mechanisms. In paper-based detections, the signals are generated either by a signal strengths amplification or surrounding noises reduction. High inaccuracy is the result of paper substrates properties due to the fact that they are random in spatial arrangements. Thus, various efforts have been made for the signal improvements such as tuning the fluid handling characteristics in the paper substrates.

For the commercial success of the biosensors, the application of compatible manufacturing technologies for large mass production is a crucial point and deterministic step [3].

Moreover, in recent years, label-free biosensors combined with innovative signal transduction methods have been proposed to push the detection limits down to femto-molar concentrations of analytes [4]. Concurrently, researchers have also been integrating sensitive and compact nano-sensors with micro-fluidics for automated sample handling. While micro-fluidics can enable portable and lab on-a-chip systems, recent theoretical and numerical calculations indicate that the effects of various fluidic integration schemes can fundamentally limit the sensors performance.

In medical diagnosis and clinical analysis sectors, the usage of nano-sensors to determine specific anatomical sites or scrupulous cell types in the human body is potentially expanding [5]. Nano-sensors provide high sensitivity and ease of miniaturization, which can help to project a new model for clinical and field-deployable analytical instruments for DNA testing, biomarker discovery, cancer diagnosis and recognition of infectious microorganisms [6].

Hence, many drawbacks still require attempting for the clinical applicability of nano-sensors. For example, the increasing need for analytical instruments requiring smaller sample volumes, reduced energy consumption, mass production, improved performance and internet connectivity have to be addressed [7].

Moreover, while the potential of biosensors in various devices is undeniable, several challenges persist in existing implementations. Conventional devices often lack the seamless integration of bio-receptors and transducers, leading to signal loss and reduced sensitivity. Moreover, the conversion of analog signals to digital form may introduce inaccuracies, hampering data interpretation. Many devices also struggle with noise interference and signal variability, compromising the reliability of collected data. Additionally, limited software capabilities often restrict users from selecting different analyte concentrations, hindering versatility in applications.

In conclusion, new diagnostic biosensors will help better diagnose and follow up diseases, wherein the implementation of novel Point-of-Care systems (POCs), easily usable by patients, and portable, or implantable devices with data transferring capabilities represents the challenge of the near future.

### Prior Art

US20130065777A1 discloses nanostructure biosensors and systems as well as methods of use thereof. Said document neither addresses nor discloses printable, portable, flexible biosensors with internet connectivity and manufacturing processes for mass production. EP 3514542 A1 discloses a detection system with a test strip formed from a flexible substrate such as paper or PET. An electrode contour is formed on the substrate and the surface of the electrode contour is modified with antibody nanoparticles. The detector includes wireless transmission means for transmitting the detection result to a cloud system. US 2009/298104 A1 discloses a subcutaneous blood glucose sensor with a flexible substrate. A data processing unit receives analyte data and operates with a wireless communication protocol such as 802.11x. The sensor includes a working electrode, a counter electrode and reference electrode.

US 2018/256083 A1 discloses a subcutaneous blood glucose sensor with a flexible substrate, showing three electrodes on a substrate that may be stacked, wherein reference is made to an 802.11x wireless communication protocol

XP 093173655 of Bariya Malika et al. referring to "Roll-to-Toll Gravure Printed Electrochemical Sensors for Wearable and Medical Devices" in ACS Nano, discloses an electrochemical biosensor with electrodes printed on a flexible PET substrate. There is a functionalized layer on top of the electrode layer. A printed circuit matrix is used for signal processing and wireless transmission to a smartphone.

XP 093173118 of Shanmugan Nandhinee Radha et al relates to "Ultrasensitive and low-volume point-of-care diagnostics on flexible strips - a study with cardiac troponin biomarkers", in Scientific Reports, discloses ZnO nanostructures on a working electrode provided with a flexible Pi membrane. The nanostructures are modified with antibodies that bind the antigen of interest, herein cardiac troponin-T - inclusion.

XP 093173140 of Wang Li et al 119/2021 relating to "A MXene-functionalized paper-based electrochemical immunosensor for label-free detection of cardiac troponin I" in Journal of Semiconductors discloses a paper electrochemical immunosensor for troponin I detection. Capture cTNI antibodies are deposited on a screen-printed electrode modified MXene nanosheets.

XP 029903405 of Jo Hunho et al relates to a "Highly sensitive amperometric detection of cardiac troponin I using sandwich aptamers and screen-printed carbon electrodes", in Talanta, Elsevier.

### Aim of the invention

In this scenario, the invention aims to overcome the main limitations of current technologies for the design and manufacturing of novel biosensors for diverse applications. The aim of the invention is thus to overcome the drawbacks, resp. shortcomings in the prior art.

A purpose of the invention is the design and fabrication of biosensors that are fully printable that may be applied in wearables, point-of-care systems, telemedicine and in general in healthcare, sports, food, industrial, public and domestic environment.

Accordingly, this invention lies on the design and production of fully printable, flexible, portable biosensors that can detect biomarkers of diseases or other conditions with high selectivity and accuracy, even in 0,01 nanograms/ml (ng/ml), with a rapid response time. Especially, a paradigm of its application is provided for early diagnosis, screening, monitoring and prevention of life-threating conditions, like heart attack. These biosensing devices may be applied to wearables notably, as well as point-of-care diagnostic systems, loT, telemedicine applications, food, pharma, cosmetics packaging, bioterrorism.

### Summary of the invention

To achieve the above objectives, a technical solution is proposed according to the present invention, wherein the biosensing device consists of a fully printable, flexible, portable, nanotechnology enabled biosensor that is biofunctionalized appropriately for detection of biomarkers of interest and quantitative measurements thereof; and an electronic device with internet connectivity for data transmission.

According to a basic embodiment of the biosensing device of the invention, there is thus proposed a biosensing device for diseases detection including for diagnoses, monitoring and screening thereof, esp. at nanoscale, comprising a nanotechnology enabled biosensing means, which is biofunctionalized for the detection of a selected set of biomarkers and quantitative measurements thereof, and an electronic device with internet connectivity for data transmission. Said biosensing device is remarkable in that it comprises all together Flexible Substrate means as well as Contacts-Electrodes means, Reference Electrode RE means, Working and Counter Electrode CE means, Dielectric means and Biofunctionalization means, which are patterned as a set of successive layers; said electrode means are arranged onto said flexible substrate means, and said biofunctionalization means detects said biomarkers and monitors same.

The biosensor is composed of electrodes deposited onto a flexible substrate and a biofunctionalization layer for detection and monitoring of diverse biomarkers including diseases, life-threating conditions like heart attack, stroke, lung embolism, diabetic hypoglycemia or hyperglycemia, infections with pathogens, allergens, air pollutants, airborne microorganisms and nanosized viruses -e.g. influenza, HIV, SARs, SARs-CoV-2-, inflammation and oxidative stress, cancer, neurogenerative diseases, trauma, bacteria and microorganisms, health and wellbeing states, vital signs, sports, food, antibiotics and drugs, agriculture, bioterrorism, cosmetics.

According to a further embodiment of the biosensing device of the invention, said biosensing device is arranged as a stack structure consisting of a superposition of said layers including said Flexible Substrate layer supporting said Contacts-Electrodes layer, Reference Electrode RE layer, Working & Counter Electrode CE layer, Dielectric layer and Biofunctionalization layer esp. in this order, which are tailored to a mutually similar peripheral shape, particularly with a substantially square or rectangular like outline, more particularly wherein said layers are made of mutually biocompatible materials.

According to a completed embodiment of the biosensing device of the invention, an insulation means is incorporated therein, advantageously consisting of an insulation layer, which is provided between said electrodes means and which is tailored to the abovementioned similar peripheral shape of said further electrodes means with said similar outline; particularly, it can be made of at least one polymer-based dielectric paste, which is applied to cover the said biosensing device.

Said insulation means resp. insulation layer, maintains electrical isolation between the various said electrodes, thereby preventing electrical contact between said electrodes, i.e. said working electrode, counter electrode, and reference electrode, thus avoiding potential merging of their functions, which allows said working electrode WE, counter electrode CE, and reference electrode RE to operate independently. This ensures accurate detection and signal transduction. The insulation layer further prevents crosstalk or leakage of electrical signals between said electrodes, which allows an accurate measurement of said biomarkers or analytes. Said insulation layer mutually isolates said electrodes, thereby contributing to preserve the integrity of the electrochemical signals generated by the interaction between said analyte or biomarkers and the biosensing device, thus enabling an accurate detection and quantification of said biomarkers.

Said insulation layer thus plays a critical role in ensuring the proper functioning of the biosensor by preventing electrical interference and ensuring independent operation of the various electrodes in the system. Specifically, the insulation layer is essential to maintain electrical isolation between the different electrodes including working electrode, counter electrode, and reference electrode. Without proper insulation, these electrodes could inadvertently come into electrical contact, leading to potential merging of their functions. This would render the device ineffective, as the electrodes are designed to operate at different potentials and perform distinct tasks. The working electrode WE, the counter electrode CE, and the reference electrode RE must operate independently to ensure accurate detection and signal transduction. The insulation layer ensures that there is no crosstalk or leakage of electrical signals between these electrodes, which is crucial for the accurate measurement of biomarkers or analytes. By isolating the electrodes, the insulation layer helps preserve the integrity of the electrochemical signals generated by the interaction between the analyte and the biosensor. This is crucial for the accurate detection and quantification of biomarkers, ensuring that the sensor provides reliable results.

The contact electrode is designed to interface directly with said reference, working, and counter electrodes. However, these three electrodes remain electrically isolated from each other due to the physical distance maintained between them. The insulation layer serves a dual purpose in this configuration: it ensures that the liquid sample interacts exclusively with said reference, working, and counter electrodes, preventing any direct contact with the contact electrode layer. Additionally, the insulation layer acts as a protective barrier, covering and shielding the three contacts of the contact electrode from unintended contact with the surrounding environment, thereby preserving the integrity and accuracy of the electrochemical measurements. This insulation allows each electrode to send and receive its own signal without affecting the others. This arrangement allows also each electrode to operate at distinct potentials as required for the device's functionality.

As to the biosensor's flexible substrate, it further amplifies its versatility, enabling comfortable and convenient usage in diverse clinical scenarios. The flexibility factor enhances patient comfort and facilitates usage across different anatomical positions. It also enables the biosensor's integration into wearables and point-of-care systems for various applications.

In terms of the biofunctionalization process of the Biofunctionalization layer, conductive nanoparticles (NPs) can be synthesized including inorganic ones, gold, silver, aluminum, platinum, palladium, graphite, silver or copper, and organic, conductive polymeric nanoparticles NPs made of glassy carbon, and conductive materials, in order to enhance the selectivity and sensitivity of the biosensing system.

The Biofunctionalization layer may comprise nanoparticles, nanotubes, diagnostic agents like antibodies or mixtures thereof. The nanoparticles can be functionalized with at least one diagnostic agent like an antibody or antibody fragment or nanobodies thereof, a receptor, a binding protein, a recombinant fusion protein, a peptide or a nucleic acid molecule or mixtures thereof that provide the recognition of the specific biomarker of interest.

In a particular embodiment of the biosensing device according to the invention, said at least one diagnostic agent comprise a first capture agent and a second capture agent, wherein the first agent is specific for the second capture agent, and the second capture agent is specific for one or more biomarkers and biomolecular targets.

A "nanoparticle" as referred herein, notably refers to a nano-plasmonic structure, nanotube, nanowire, nanosphere having dimensions that vary from about 2 nm or less to about 1800 nm or less. The shape may be spherical, as well as discoid, hemispheric, rectangular, polygonal, triangular, square, cuboid, pyramidal, tubular and/or wire form.

According to another aspect of the invention, a method of making a sensor, esp. a biosensor, is provided that includes a fully printable, flexible, portable cardiac biosensor for the early diagnosis of life-threating conditions like heart attack in patients with chest pain and the transmission of the qualitative and quantitative measurements of the troponin cardiac biomarker to the nearest doctor's office, ambulance, hospital for quick therapeutic intervention.

Accordingly, there is also proposed according to the present invention a robust, reliable method for design, development and mass production of nanotechnology enabled sensing devices exhibiting high selectivity and specificity for an early diagnosis, screening of life-threating conditions like heart attack and its internet connectivity for data transfer and streamlining patient care.

While the primary focus of this aspect of the embodiment is the detection of troponin levels in patients from only a drop of blood, the biosensor's advanced capabilities extend beyond, offering a holistic approach to other diseases diagnosis, monitoring, prevention as well. The accurate and rapid results enable medical professionals to assess a patient's health comprehensively, aiding in informed decision-making.

According to a basic embodiment of the invention, there is proposed a pioneering method for the fabrication of biosensors using a Roll-to-Roll manufacturing process, which leverages the benefits of printing technology, precise annealing procedures, and in-line characterization techniques. Printing techniques, including screen printing, gravure, inkjet, laser printing and the like can be applied. The biosensor development, in large scale using a roll-to-roll system, process begins by employing printing to deposit successive layers of biocompatible materials onto a continuous flexible substrate roll. The use of a roll-to-roll machine for biosensor fabrication represents a significant advancement over traditional methods. This scalable approach not only boosts production efficiency but also enables cost-effective manufacturing, making the biosensor solution accessible to a wider range of applications.

According to a preferred embodiment of the method of the invention, it further comprises a laser ablation step in the Roll-to-Roll process to precisely pattern the conductive electrode layers onto the flexible substrate, wherein the laser ablation process allows high-precision scribing of micro- to nano-scale features, further minimizes heat-affected zones for material integrity, and enables real-time beam adjustments to ensure uniformity and reduce production defects. Laser ablation is thus a key element in the method of the invention.

The design of the biosensor and the selection of materials among notably polymers, plastics, substrates, nanoparticles and compounds is made in line with the specific application.

The control of the thickness, architecture, structural and physicochemical properties of the engineered nanomaterials is achieved upon the fabrication method, e.g. alterations in printing parameters, material and ratios, in line with ultrasensitive measurements and monitoring by Integrated Characterization Tools.

The biosensor manufacturing process of the invention integrates an array of cutting-edge in-line and ex-situ characterization tools. Ellipsometry spectroscopy, Raman Spectroscopy, Eddy Current, Atomic Force Microscopy (AFM), Dynamic Light Scattering (DLS), UV-Vis Spectrophotometry, and Voltammetry synergistically monitor the quality and functionality of each production step. This robust quality control mechanism ensures consistently high-quality biosensors, reducing the likelihood of manufacturing defects and optimizing performance.

According to a further embodiment of the invention, there is proposed the design and fabrication method of a cardiac biosensor for early diagnosis and risk stratification of cardiovascular disease event like heart attack. The cardiac biomarker to be detected is cardiac troponin, but may be also CPK-MB, as well as natriuretic peptides (NPs), C-Reactive Protein (CRP), Copeptin, Cardiac Myosin-Binding Protein C (cMyC), Heart-Type Fatty Acid Binding Protein (Hfabp), Endothelial Cell Related Biomarkers and the like.

According to a yet further embodiment of the invention, an innovative biosensor signal reader and analysis device bridges the gap between the bio-receptor and accurate data interpretation. By establishing covalent bonding between bio-receptors and transducers, converting analog signals to digital, and enabling precise selection of analyte concentrations, this device revolutionizes biosensor technology. The integration of cutting-edge hardware and software ensures real-time data collection, analysis, and visualization, making it an indispensable tool for various applications in healthcare, diagnostics, and research.

This invention further relates to the functionalization process of the biosensor to enhance their ability to detect disease biomarkers at an early stage, at very low concentrations, quickly for primary therapeutic intervention.

To summarize, the advantages of the embodiment are synthesized hereafter:
i) a high sensitivity and selectivity of the biosensors due to the fine-tuned thickness, structural properties, architecture of its nanoscale dimensions of layers and novel biofunctionalization enabling the detection of ultra-low levels of biomarkers of interest, even in 0,01 ng/ml;
ii) fully printable, light-weight biosensors on flexible substrates that provide them with flexibility and portability:
iii) qualitative and quantitative results from small samples-only drops of blood or other biological samples/analytes for diagnosis, screening, monitoring and prevention of diseases notably;
iv) significant information on blood properties and vulnerability for cardiovascular disease risk stratification of individuals;
v) rapid diagnosis with very quick time response even < 2 minutes facilitating timely medical intervention and improved patient outcome;
vi) the unprecedented use of Roll-to-Roll Printing Technology that enables mass production and cost-effective manufacturing of the biosensors;
vii) Cutting-edge in-line and ex-situ characterization tools for robust quality control of the biosensors during manufacturing to ensure their optimizing performance, and
viii) telemedicine application thanks to the capability of the biosensor for internet connectivity and data transferring for streamlining patient care.

The applications of the device of the invention comprise wearables, biomedical, personalized medicine, point-of-care diagnostics, predictive tools, diagnosis, screening, risk assessment and prognosis of diseases, sports, athlete safety monitoring, injury management, performance and fitness optimization, remote vital signs, health or recovery monitoring, personalized patient monitoring, food processing applications, agriculture, pharmaceuticals, research and drug development, bioterrorism, fine chemicals, flavor, fragrance, cosmetics, implantable devices, biomedical devices, medical equipment.

Said document EP3514542 yet discloses an impedance chip detection system for biological testing. Such sensors quantitatively measure changes in impedance, whereas the biosensor according to the invention measures square wave voltammetry-different method of analyte measurement. Moreover, it is a different type of biosensor with a different architecture. According to the invention, there is neither absorbing layer in the device according to the invention, nor airlaid paper, nor adhesive, nor solidified, nor UV preparation. Instead, the device according to the invention is a three- electrode detection system whereas said document depicts a two-electrode system notably in Figure 6.

The abovementioned document XP 093173655 depicts Roll-to-Roll Gravure Printed Electrochemical Sensors for Wearable and Medical Devices". The method according to the invention is not limited to gravure printing however, but it consists of a Roll-to-Roll printing method notably involving screen printing, flexography printing or inkjet printing. Moreover, the biosensor produced by gravure printing disclosed in said document, has a design and architecture that is different from the device according to the invention.

Said document XP 093173140 depicts a portable paper-based electrochemical immunosensor constructed by wax printing and screen-printing, i.e. with the need of the combination of two methods. This is in contrast with the invention, based on a different method of fabrication of the device, a different architecture, having no paper based biosensor, but instead diagnostic nanoparticles as a part of the biosensor according to the invention with digital capabilities.

Document US2009/298104 discloses Ag/AgCl-based reference electrodes designed for use in long-term amperometric sensors for in vivo glucose monitoring. As to the production method, it focuses on membrane and coating technologies that protect the Ag/AgCI reference electrode. It measures the electrochemical current generated from the enzymatic oxidation of glucose, which produces hydrogen peroxide that is subsequently oxidized at the working electrode. This sensor is sensitive to glucose concentrations, but its selectivity is primarily achieved by membranes that limit the access of interferents to the sensing area The production method is thus different from the invention, as well as the analyte detection. As to data transmission, this document discloses a data monitoring system for in vivo glucose levels, where data is transmitted from the sensor to a receiver unit, such as a handheld device or external monitor. However, it does not mention integration with loT or modern telemedicine platforms.

Document US2018/256083 discloses temperature-independent analyte sensors that ensure stability and accuracy in vivo. The core technology is a temperature-independent membrane, particularly for glucose sensing, which limits the variability in readings due to temperature fluctuations. However, this document does not specifically disclose the manufacturing process in detail, but instead focuses more on the functional aspects of the analyte sensors and the technicalities of temperature compensation in glucose measurement. This document deals with a more rigid electrochemical sensor design, focusing on achieving temperature independence via membranes that can stabilize glucose measurements at varying temperatures. This document focuses on in vivo glucose monitoring with minimal mention of data transmission features. The system is designed for continuous monitoring but does not integrate !oT or telemedicine as a core feature. It refers to general troponin measurement, but it does not specify which troponin.

Another paradigm of such a sensing device is to transfer the data to the nearest doctor's office, ambulance, or hospital for quick, effective treatment of the patient. The biosensor's exceptional accuracy complemented by an impressively rapid response time and data transmission capability ensures timely medical attention, minimizing the risk of complications and streamlining patient care. Thus, the present embodiment circumvents the rapid diagnostic tests and point-of-care systems used in clinical practice that lack data transferring for decision making.

According to another aspect of the invention, this sensor, such as a biosensor, is provided herein that can be used as a valuable screening, predictive tool from medical doctors to assess cardiovascular risk, to predict life threating conditions and to plan tailored risk factor intervention in high risk individuals for cardiovascular disease (CVD).

Further particularities and characteristics of the invention are defined in the further subclaims.

The device and method of the invention is further illustrated by appended drawings, in which further details are explained in more detail in the following description in some embodiments of the invention with reference to the attached drawings.

### Brief description of the drawings

**Fig. 1a** is a schematic representation of the structure of a first embodiment of a biosensor according to the invention.
**Fig. 1b** depicts a schematic representation of the Biofunctionalization Biosensor device structure according to the invention as achieved by nanoparticles with antibodies for disease biomarkers detection.
**Fig. 1c** is a schematic representation of the structure of a second embodiment of a biosensor according to the invention.
**Fig. 2a** depicts a schematic representation of the structure of the biosensor electronic device according to the invention.
**Fig. 2b** presents a schematic representation of the external surface of the biosensor electronic device according to Fig. 2a of the invention, and its components.
**Fig. 3a** depicts a schematic representation of Roll-to-Roll printing method for large scale production of the biosensor according to the invention;
**Fig. 3b** depicts a schematic representation of laser ablation as a key step in the Roll-to-Roll process according to the invention, to precisely pattern the conductive electrode layers onto the flexible substrate.
**Fig. 4**a depicts an Atomic Force Microscopy analysis of gold Nanoparticles (GNP) that can be applied for further functionalization of the body of the invention;

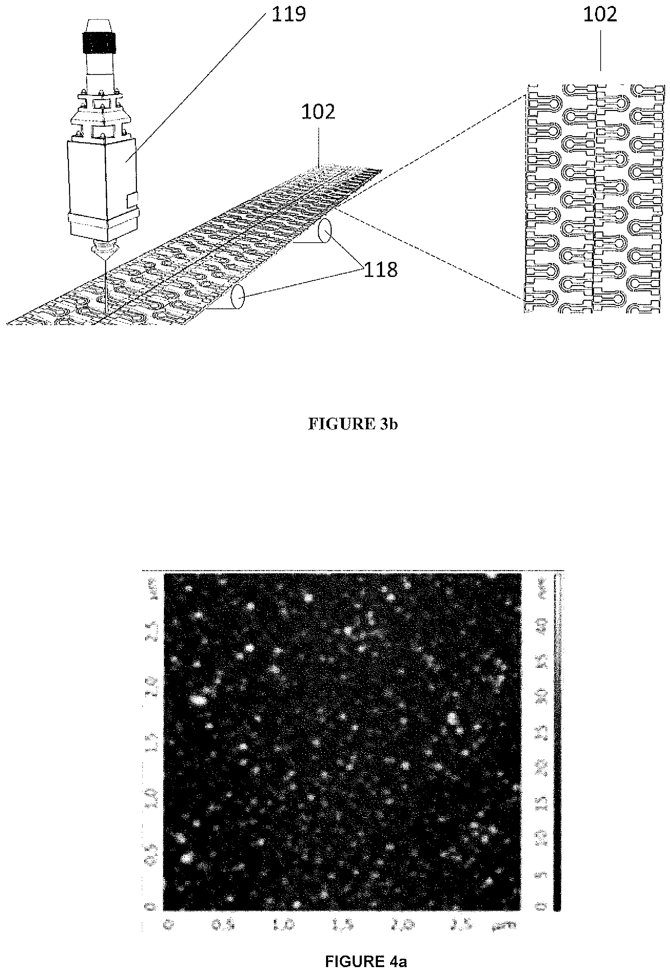

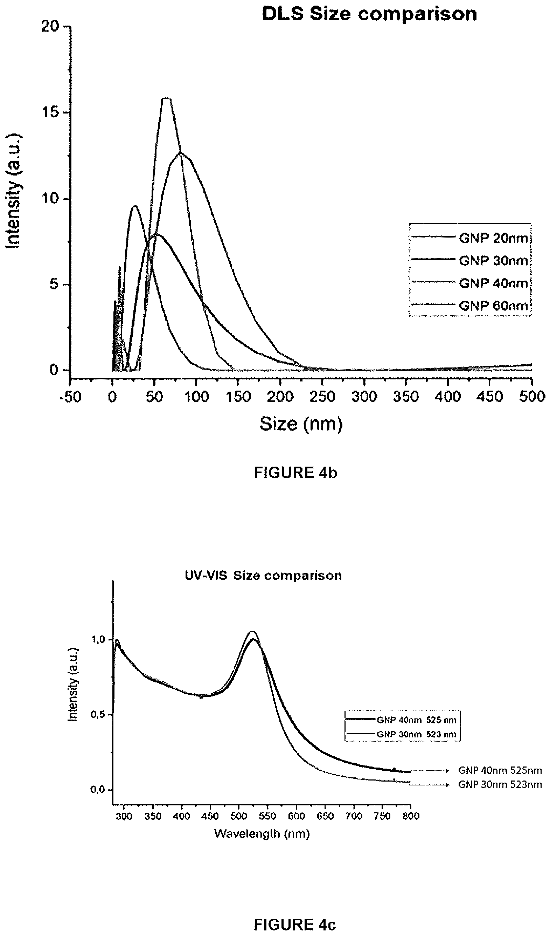

**Fig. 4**b depicts a graph of Dynamic Light Scattering analysis that measures the hydrodynamic diameter of GNPs used in biofunctionalization step of the biosensor;
**Fig. 4**c depicts a graph of UV-Vis spectroscopy analysis for nanoparticle size comparison and characterization.

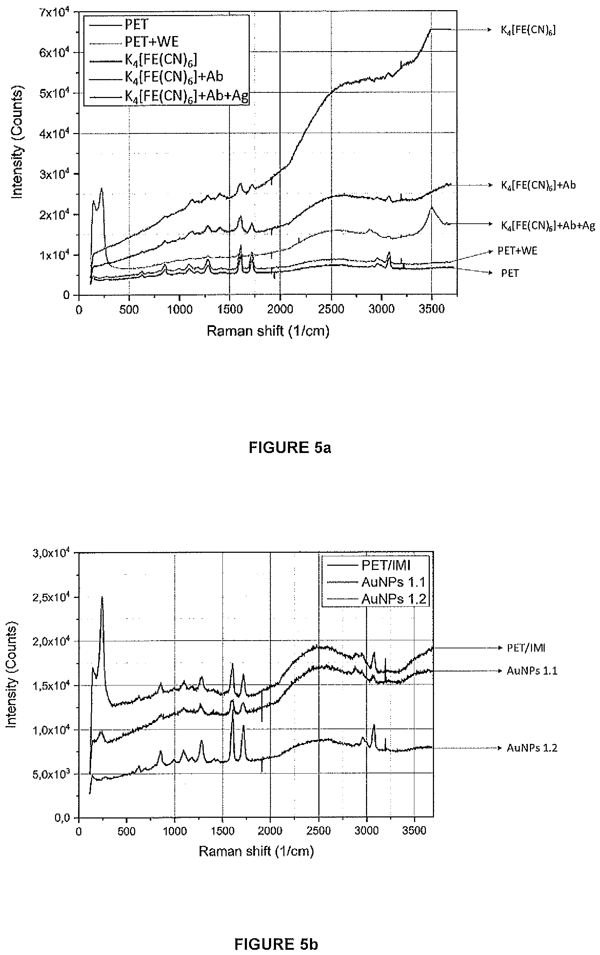

**Fig. 5a** represents a graph of Raman Spectroscopy analysis of all layers of the biosensors according to the invention;
**Fig. 5b** represents a graph of biofunctionalization layers with different concentration.

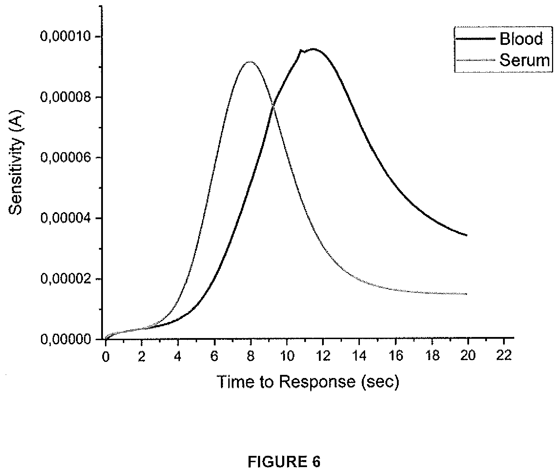

**Fig. 6** represents a graph of the time response of the biosensor according to the invention in samples of human blood and serum.

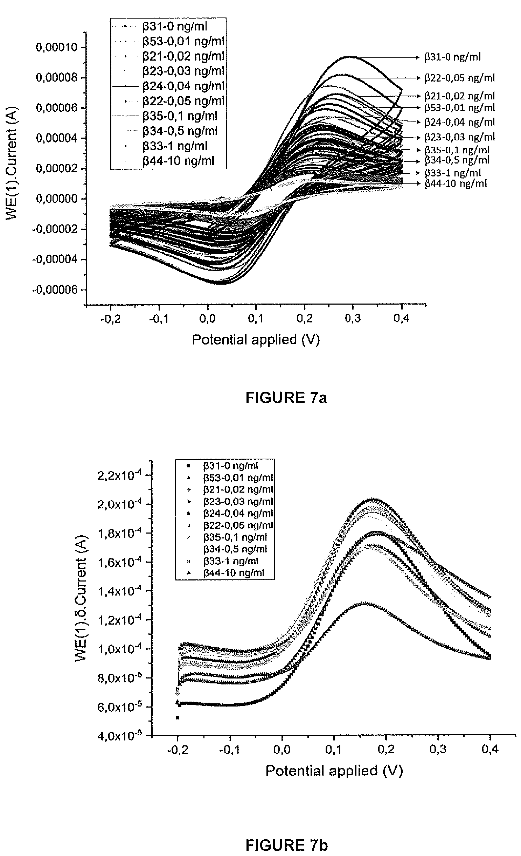

**Fig. 7a** illustrates a graph of measurements of concentrations of antigen in biofunctionalization step of the biosensor according to the invention with Cyclic Voltammetry, and
**Fig. 7b** illustrates a graph of measurements of concentrations of antigen in biofunctionalization step of the biosensor according to the invention with Square Wave Voltammetry;

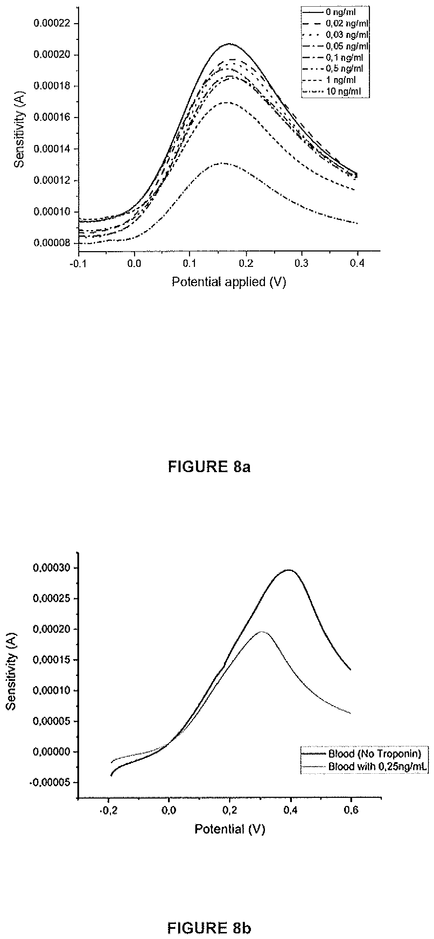

**Fig. 8a** illustrates a graph of the measurements of the sensitivity versus potential of the cardiac troponin biosensor of a further embodiment of a biosensor according to the invention that detects the cTnT antigen in serum at different concentrations, whereas
**Fig. 8b** illustrates the measurements of the sensitivity versus potential of said cardiac troponin biosensor that detects the cTnT antigen in whole human blood;

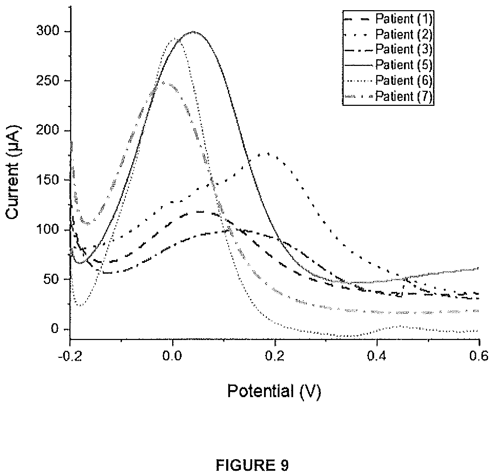

**Fig. 9** illustrates a graph of the measurements of the sensitivity versus potential of the said cardiac troponin biosensor according to the latter further embodiment of the invention in blood samples of patients, wherein the data derived can be used not only for diagnosis of a heart attack but also for screening of patients with high cardiovascular risk.

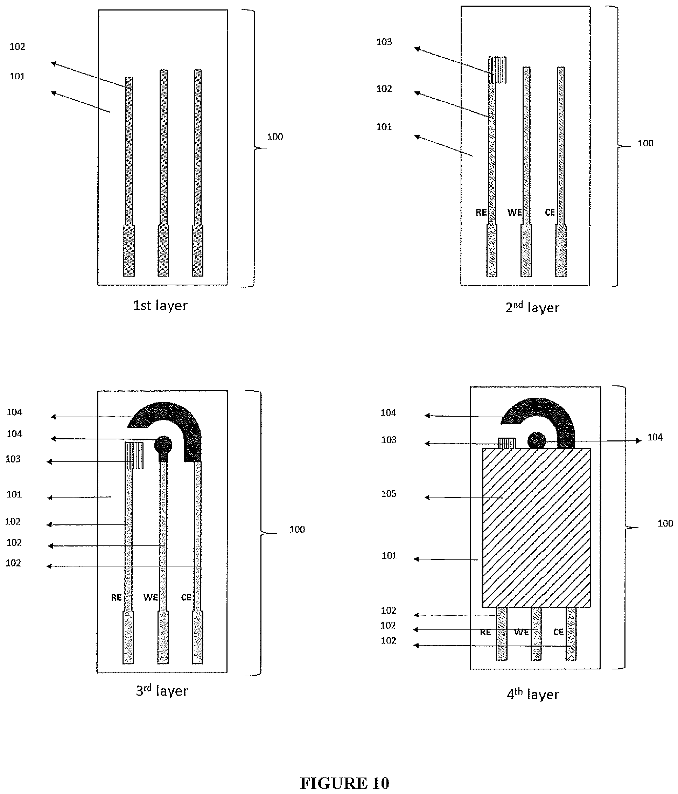

**Fig. 10** is a schematic representation of the layer structure of a preferred embodiment of a biosensor according to the invention set out for its 4 layers.

### Description

The invention relates to a fully printable, flexible, portable and light-weight biosensor with high selectivity and specificity to detect and measure biomarkers of diseases and other conditions for the case of the following exemplary embodiments:
a first embodiment consists of the design of an advanced biosensing *system* device for early diseases diagnosis and monitoring comprising a fully printable, flexible, nanotechnology enabled biosensor that is appropriately biofunctionalized for the detection of biomarkers of interest; as well as an Electronic device with internet connectivity for transferring the qualitative and quantitative measurements of the specific biomarker to the nearest data center.

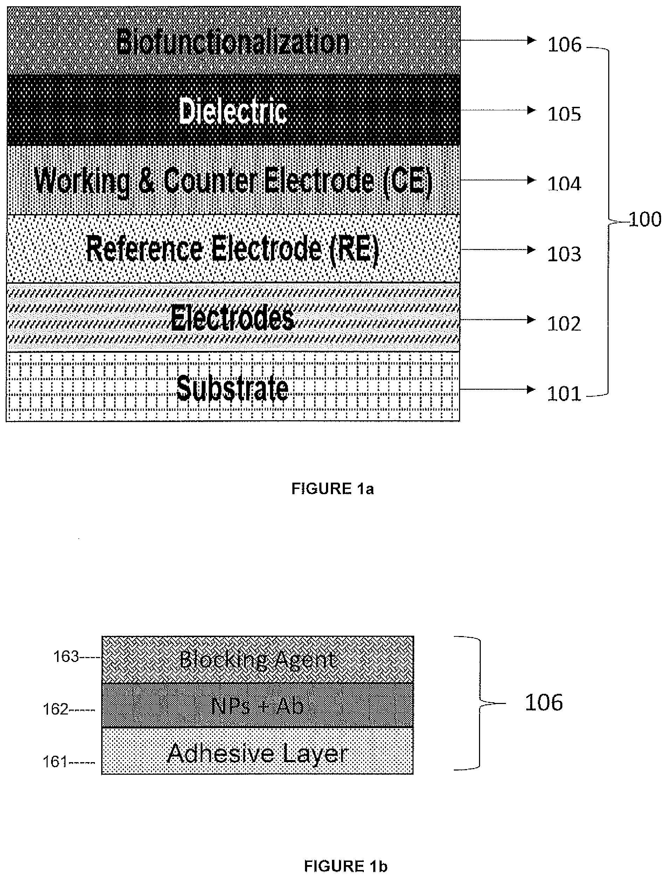

Fig. 1a shows a first structure of the biosensor 100 comprising:
a) Electrodes 102 deposited onto a flexible substrate 101,
b) a Reference Electrode (RE) 103,
c) Working and Counter Electrode (CE) 104,
d) Dielectric means 105,
e) a Biofunctionalization layer 106 and
insulation layer means.

A preferred example of arrangement thereof consists of a stack structure of the biosensor device as depicted in Fig. 1a showing a superposition of Flexible Substrate 101 /Contacts-Electrodes 102 /Reference Electrode (RE) 103 [Working & Counter Electrode (CE) 104 /Dielectric 105 /Biofunctionalization 106 esp. successively in said order. All these layers including the metal electrode 102 are fully printed by a screen-printing method under ambient conditions. The printing process consists of consecutive printings of different materials in a standard order. Those layers, in printing order, are: Contacts, Reference Electrodes 103, Working and Counter Electrodes 104 and Dielectric Layer 105. Thermal annealing is applied to each functional layer after the printing process.

Said flexible substrates 101 are made of materials notably consisting of Polyimide (PI). And also Polyethylene Naphthalate (PEN), Polydimethylsiloxane (PDMS), Polycarbonate (PC), Polyethylene Terephthalate Glycol (PETG), Olefin Copolymer (COC), Polyester (PET); also Flexible Glass, Paper-Based Substrates, woven and non-woven fabrics, Polymer Blends. The flexibility of the substrate 101 provides the superior advantage to biosensor allowing integration into wearables, point-of-care systems and other whereas the material selection depends on the application.

The Biosensor Contact Electrodes 102 are made of silver paste notably; but also Graphene, Silver Nanowires, Conductive Polymers like polyaniline, polypyrrole, and PEDOT:PSS(poly(3,4-ethylenedioxythiophene):poly(styrenesulfonate)), indium Tin Oxide (ITO) Nanowires, Metal Nanoparticles, esp. gold as well as silver ones, Metal Oxides especially Tin oxide or zinc oxide, Carbon-based inks, such as carbon black or graphite inks, Flexible Metal Film especially copper or aluminum.

The Reference Electrode RE 103 is fabricated from silver/silver chloride (Ag/AgCl) paste with a sheet resistance of less than 100 Ω/sq. Said reference electrode 103 is incorporated essentially to provide support in the transduction of electrical signals. The accuracy of the reference electrode 103 is essential in electrochemical sensing as it provides support in the transduction of electrical signals. Further materials used for the Reference Electrode (RE) 103 notably consist of Silver/Silver Chloride (Ag/AgCl) paste, silver nanoparticles and silver nanowires, Ag/AgCI nanoparticles, Graphene Oxide (GO) Ink mixed with Ag/AgCI nanoparticles, noble metals esp. gold or platinum, as well as their metal oxide nanoparticles, metal oxide inks esp. ruthenium oxide (RuO₂), iridium oxide (IrOx) or Titanium Oxide (TiO₂), carbon Inks, esp. graphite, carbon, or graphene oxide (GO) paste and their nanotubes, Copper/Copper Sulfate paste (Cu/CuSO₄), conductive polymers esp. polyaniline, PEDOT:PSS, Polypyrrole.

The working and counter electrodes 104 are comprised of conductive carbon sensor paste, each with a sheet resistance of less than 75 Ω/sq.

The insulation between the electrodes of the system is achieved using various polymer-based dielectric pastes, which are applied to cover the device. Each dielectric paste has different properties, such as durability in chemicals, durability in annealing, and hydrophobicity.

Further materials used for Working (WE) and Counter Electrodes (CE) 104 may consist of carbon inks esp. graphite, carbon or graphene oxide (GO) paste and their nanotubes, noble metals esp. gold, platinum, silver, copper and palladium and their nanoparticles, conductive polymers esp. such as polyaniline, PEDOT:PSS and Polypyrrole, metal oxide nanoparticles especially titanium dioxide (TiO₂), indium tin oxide (ITO), and tin oxide (SnO₂).

The contact electrode 102 is designed to interface directly with said reference, working, and counter electrodes. However, these three electrodes remain electrically isolated from each other due to the physical distance maintained between them. The insulation layer serves a dual purpose in this configuration: it ensures that the liquid sample interacts exclusively with the reference, working, and counter electrodes, preventing any direct contact with the contact electrode layer 102. Additionally, the insulation layer acts as a protective barrier, covering and shielding the three contacts of the contact electrode 102 from unintended contact with the surrounding environment, thereby preserving the integrity and accuracy of the electrochemical measurements. This insulation allows each electrode to send and receive its own signal without affecting the others. This arrangement allows also each electrode to operate at distinct potentials as required for the device's functionality as shown in Figure 10.

The materials for Dielectric 105 comprise a diversity of inks like Gray Dielectric Ink, yet possibly also polymers like polyimides, polyethylene, polypropylene, and polyvinylidene fluoride (PVDF), barium titanate (BaTiO₃), aluminum oxide (Al₂O₃), zinc oxide (ZnO), *or* other ceramic materials with high dielectric constants, dielectric powders mixed with binders, crosslinkers, solvents and curing agents, glass-ceramic powders mixed with binders, polymer-ceramic composites, silicone-based dielectrics, Lead Zirconate Titanate (PZT) paste, dielectric elastomers, ceramic-polymer hybrid materials.

The fine-tuning of printing parameters, printing speed, temperature, humidity, time, thermal annealing parameters in line with the selection of the suitable materials, materials ratio and properties determine the efficacy of the biosensor 100.

The Biofunctionalization Biosensor device structure 106 as achieved by nanoparticles with antibodies for disease biomarkers detection like myocardial infarction is shown in Fig. 1b.

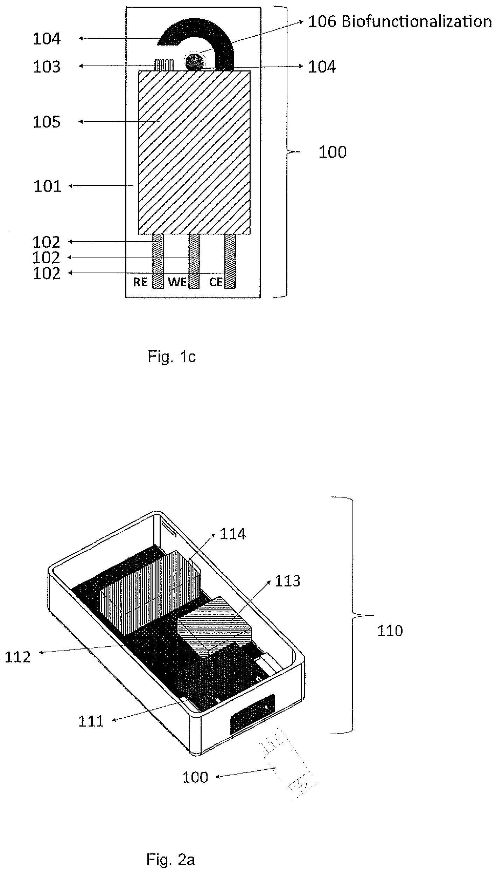

Fig. 1c shows the structure of a further embodiment of the biosensor stripe 100 and its components involving Electrodes 102 onto flexible substrate 101, Reference Electrode (RE) 103, Working and Counter Electrode (CE) 104, Dielectric 105, and Biofunctionalization layer 106.

Fig. 2a shows the structure of the biosensor electronic device 110 that can read the biochemical signal of the biosensor stripe 100 and its internal surface and parts composed of a Connector for Flexible Flat Cables (FFC)1 Flexible Printed Circuits (FPC) 111, a Printed Circuit Board (PCB) 112, Electronic Components 113, Electronic Connection Parts 114 Micro-USB or Type-C, Bluetooth module, Wi-Fi module.

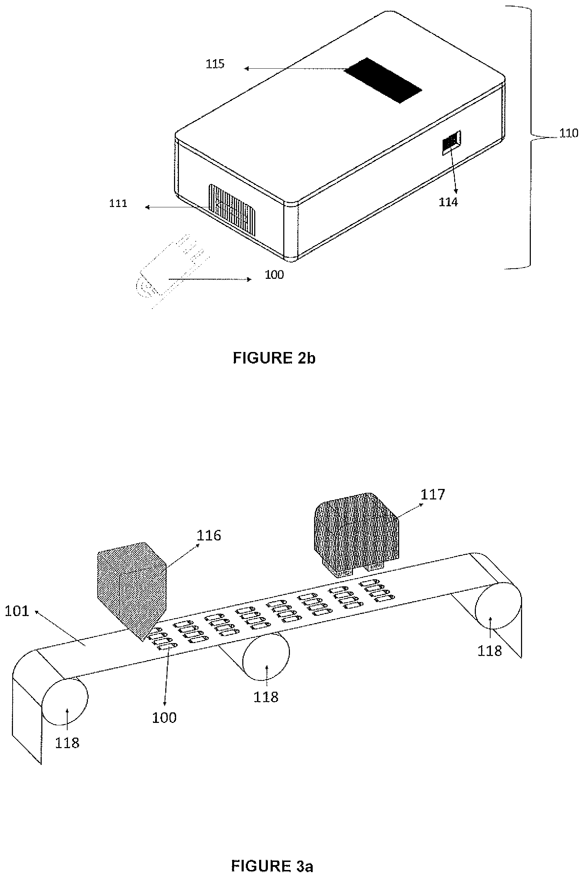

Fig. 2b shows the external surface of the biosensor electronic device 110 with its components consisting of a Monitor display 115 like Touchscreen, Touchscreen, Liquid-Crystal Display (LCD), in-plane switching monitor (IPS), Electronic Connection Parts 114 can be Micro-USB or Type-C, Bluetooth module, Wi-fi module and an FFCIFPC Connector 111.

The present invention also provides a robust method for design and development of a pioneering method for the mass production of biosensors using a Roll-to-Roll printing process as shown in Fig. 3 for large scale production of the biosensor stripes 100 deposited onto flexible substrates 101. The Roll-to-Roll machine is elaborated by cutting-edge in-line and ex-situ characterization tools for biosensor quality control 116, 117. Rolls 118 of the Roll-to-Roll system that drive the substrate and biosensor layers for printing and characterization, are also represented.

Said Roll-to-Roll manufacturing process combines the advantages of printing technologies with in-line characterization techniques for real time quality control of the engineered biosensors. The Roll-to-Roll biosensor production process is in large scale started by employing printing to deposit successive layers -even at nanoscale size- of biocompatible materials onto a continuous flexible substrate roll. The fabrication of biosensor layers on flexible substrates 101 can also be made by printing technologies, such as screen printing, as well as gravure, inkjet, laser printing and flexography printing notably.

The method according to this invention thus addresses the drawbacks of traditional methods as it enables mass production, cost-effective manufacturing of the flexible, light-weight biosensors for a wide range of applications.

The control over the thickness, architecture, structural, and physicochemical properties of engineered nanomaterials can be realized through various fabrication methods. For instance, adjustments in printing parameters, such as the drum thickness in roll-to-roll screen printing or the frame thickness in sheet-to-sheet screen printing, can influence these properties. Another crucial factor is the pattern of each layer, which ultimately defines the shape of the final biosensor. Moreover, the weave dimension of the drum or frame, along with the materials used at each production step, including their proportions and concentrations, significantly impact the layering of biosensors.

Various characterization techniques, both in situ and ex situ, are available to assess and ensure the quality of these biosensors. In-line characterization tools suitable for roll-to-roll techniques encompass Raman Spectroscopy, Ellipsometry, and Eddy Current methods. These techniques enable real-time monitoring of the printing process and the biosensors' electrodes during screen printing. Ex situ characterization techniques, such as Atomic Force Microscopy, UV-Vis Spectrophotometry, Dynamic Light Scattering, Cyclic and Square Wave Voltammetry, are indispensable for quality control and for assessing the functionality of the final biofunctionalized biosensors. An Atomic Force Microscopy analysis of the gold Nanoparticles (GNP) applied for further functionalization of the body of the invention with scan size 3×3µm is shown in Fig. 4a. A Dynamic Light Scattering analysis that measures the hydrodynamic diameter of said gold nanoparticles GNPs used in biofunctionalization step of biosensors is shown in Fig. 4b, whereas an UV-Vis spectroscopy analysis for nanoparticle size comparison and characterization is shown in Fig. 4c.

Thoroughly characterizing the structural properties of engineered systems is imperative to achieve their intended functionality.

In order to further enhance the biosensor's performance, a biofunctionalization procedure is conducted after the electrodeposition process as shown in Fig. 1b depicting a schematic representation of the Biofunctionalization Biosensor device structure as achieved by nanoparticles with antibodies for disease biomarkers detection like myocardial infarction, whereas Fig. 1c represents a the structure of the biosensor stripe and its components involving Electrodes onto flexible substrate, Reference Electrode (RE), Working and Counter Electrode (CE), Dielectric, and Biofunctionalization layer.

This procedure involves the deposition of several bio-layers on the working electrode 104, starting with an adhesive layer 161 which acts as a transport layer for the charge carriers. This adhesive layer 161 may be ionic liquid crystal, like Potassium ferrocyanide (K4[Fe(CN)6]), methylene blue, thionine and quinones. The next layer 162 is a homogenous compound made of nanoparticles (NPs) esp. like gold ones and Antibodies (Ab), which are responsible for detecting the addition of antigen on the working electrode 104 surface. A diversity of nanoparticles (NPs) can be used like inorganic ones like gold, silver, magnetic NPs, such as iron oxide ones, semiconductor quantum, carbon nanotubes, liposomes, metal oxide NPs like zinc oxide or titanium dioxide, polymeric NPs, silica ones, The NPs can be functionalized with diagnostic agent like an antibody or antibody fragment thereof, a receptor, a recombinant fusion protein, a peptide or a nucleic acid molecule that provide the recognition of the targeted specific biomarker.

The control over the size, shape, surface charge, materials, density, architecture, pattern of NPs and biofunctionalization process determines the selectivity and specificity of the engineered biosensor.

Finally, a layer of blocking agent 163 like Glycine, Mercaptoundecanol (MCU), Polyethylene glycol (PEG), Bovine serum albumin (BSA) is added to enhance the sensor's sensitivity and decrease false signals.

The sensing principle of the electrochemical biosensor was based on the voltametric inhibition peak of the K₄[Fe(CN)₆] ionic organic molecule deposited on the sensor's surface, which is possibly attributed to the azo group conjugated to the heterocycles of the K₄[Fe(CN)₆] structure when Ab molecules bind to the electrode surface. The binding was achieved with the use of NPs, where the respective antigen that needs to be detected is immobilized onto the sensor's surface by specific antibody-antigen molecular interactions.

The synthesis of gold nanoparticles used in the biofunctionalization process according to Fig. 1b is achieved using the Turkevich method. This method involves the reduction of gold ions to form stable, monodisperse, and spherical gold nanoparticles. The size and shape of the nanoparticles can be controlled by adjusting the concentration of the gold ions and trisodium citrate, as well as the reaction time and temperature.

A schematic representation of the Biofunctionalization Biosensor device structure as achieved by nanoparticles with antibodies for disease biomarkers detection is depicted in Fig. 1b.

The structure of a flexible, biosensor stripe that can detect the biomarkers of a disease or other conditions and its components involving Electrodes onto flexible substrate 101, Reference Electrode (RE) 103, Working and Counter Electrode (CE) 104, Dielectric 105, and Bio-functionalization layer 106 is shown in Fig. 1c.

According to an additional aspect of the invention, a meticulously designed biosensor electronic device is fabricated, capable of deciphering intricate biochemical signals from the biosensor stripe 100. The internal architecture of the device showcases its sophisticated components, notably the Flexible Flat Cables (FFC)/ Flexible Printed Circuits (FPC) 111 Connector, seamlessly integrating the biosensor strip for accurate signal processing. The heart of this device lies within the intricately crafted Printed Circuit Board (PCB) 112, housing Electronic Components such as Central Processing Units (CPU), Extra Sensory Perception Unit (ESP), Filters, Analyzers, Gyroscopic module, Global Positioning System (GPS). These components work in harmony to interpret the biosensors signals, ensuring precise and reliable data analysis. The device also incorporates Electronic Connection Parts 114 like Micro-USB or Type-C, Bluetooth module, and Wi-fi module, enhancing its connectivity and functionality as represented in Fig. 2a. showing the structure of the biosensor electronic device 110 that can read the biochemical signal of the biosensor stripe and its internal surface and parts composed of a Connector for Flexible Flat Cables (FFC)1 Flexible Printed Circuits (FPC), a Printed Circuit Board (PCB), Electronic Components, Electronic Connection Parts Micro-USB or Type-C, Bluetooth module, Wi-Fi module. This intricately engineered biosensor electronic device 110 exemplifies cutting-edge technology at its finest, paving the way for advanced biosensing applications.

An external view of the biosensor electronic device highlighting its user-friendly interface and streamlined components is depicted in Fig. 2b showing the external surface of the biosensor electronic device 110 and its components, consisting of a Monitor display 115 like Touchscreen, Touchscreen, Liquid-Crystal Display (LCD), in-plane switching monitor (IPS), Electronic Connection Parts 114 can be Micro-USB or Type-C, Bluetooth module, Wi-fi module and an FFC/FPC Connector. The device 110 boasts a Monitor display 115 that encompasses various options such as Touchscreen, Liquid-Crystal Display (LCD), in-plane switching monitor (IPS), ensuring clear and vibrant visual representation of the interpreted data. The device's seamless integration is facilitated by the FFCIFPC Connector 111 bridging the biosensor strip 110 and the electronic components with precision. Electronic Connection Parts 114 like Micro-USB or Type-C, Bluetooth module, and Wi-fi module provide versatile connectivity options, allowing the user to transfer and access data effortlessly. This comprehensive design not only ensures efficient functionality but also prioritizes user experience, making it an indispensable tool in the realm of biosensor technology.

A yet further example consists of a design and development of cardiac biosensor for heart attack diagnosis and monitoring at an early stage made of: i) Fully printable, flexible, nanotechnology enabled biosensor that is appropriate biofunctionalized for detection of troponin biomarker of a heart attack, ii) Electronic device with internet connectivity for transferring the quantitative measurements of the biomarker to the nearest ambulance, hospital for quick therapeutic intervention.

In clinical practice, troponin is the biomarker of choice for the detection of cardiac injury and infarction size that underlies the onset of myocardial infarction and potential cardiac arrest [8,9]. Thus, the troponin measurements in patients who undergo chest pain are vital for their effective treatment based on the guidelines published by the European Society of Cardiology or American Heart Association [10]. The treatment depends on when the symptoms started and how soon the patient can access treatment like primary percutaneous coronary intervention. The American Heart Association has released Heart and Stroke Statistics - 2022 Update [11]. According to the report, cardiac arrest remains a public health crisis. There are more than 356.000 out-of-hospital cardiac arrests (OHCA) annually in the U.S., nearly 90% of them fatal. The incidence of EMS-assessed non-traumatic OHCA in people of any age is estimated to be 356.461, or nearly 1000 people each day. Survival to hospital discharge after EMS-treated cardiac arrest languishes at about 10%. As heart attack is the main cause of cardiac arrest.

The biosensor of the invention offers unparalleled sensitivity, enabling the detection of ultra-low levels of troponin (0,01 ng/ml), even surpassing human detection rates. This level of precision is pivotal for diagnosing heart problems at an early stage, facilitating timely medical intervention and improved patient outcomes.

In an analogous manner to the previously described biosensor's stack structure 100, the cardiac biosensor is delineated as Flexible Substrate/ Contacts-Electrodes /REIWE/CE/Dielectric/Biofunctionalization, adhering to a well-defined configuration as shown in Fig. 1a. The Biofunctionalization layer 106 is made of conducting nanoparticles especially like gold ones with Abs against troponin biomarker.

The biofunctionalization layer 106 comprises nanoparticles, nanotubes, antibodies or mixtures thereof. The conducting nanoparticles typically should be made esp. from gold, NPG - yet as well as aluminum, platinum, palladium, graphite, silver or copper, glassy carbon, conductive materials - and their size, surface charge and geometry depend on the specific biosensing application.

A "nanoparticle" as described herein, refers to but not limited to a nanoplasmonic structure, nanotube, nanowire, nanosphere having dimensions that vary from about 2 nm or less to about 1800 nm or less. The shape may be spherical, discoid, hemispheric, rectangular, polygonal, triangular, square, cuboid, pyramidal, tubular, wire form.

The NPs can be functionalized with diagnostic agent like an antibody or antibody fragment or nanobodies thereof, a receptor, a binding protein, a recombinant fusion protein, a peptide or a nucleic acid molecule or mixtures thereof that provide the recognition of the specific biomarker.

The cardiovascular biomarker to be detected may be cardiac troponin notably, as well as CPK-MB, natriuretic peptides (NPs), C-Reactive Protein (CRP), Copeptin, cardiac Myosin-Binding Protein C (cMyC), Heart-Type Fatty Acid Binding Protein (Hfabp), Endothelial Cell Related Biomarkers like Endothelial cell-specific molecule 1 (ESM-1), Platelet Related Biomarkers Mean like platelet volume (MPV) and beta-thromboglobulin (beta-TG), Suppression of Tumorigenicity 2 (ST2), Matrix metalloproteinases (MMP), Cystatin C (cys-C), miRNAs, miRNA-208 and miRNA-499, Long Noncoding RNAs (IncRNAs), Sirtuin (SIRT), Triggering Receptor Expressed on Myeloid Cells (TREML), Growth-Differentiation Factor-15 (GDF-15), Pregnancy-Associated Plasma Protein-A (PAPP-A), hosphatidylinositol 3-phosphate kinase (PIK3C2A) and the protein arginine methyltransferase 5 (PRMT5), YKL-40, inflammatory Biomarkers, Soluble CD40 Ligand (sCD40L), Galectin-3 (Gal-3), Lipoprotein-associated phospholipase A2 (Lp-PLA2), interleukin-37 (IL-37), S-100 protein, Neuron Specific Enolase.

The present invention also relates to a pioneering method for the fabrication of biosensors using a Roll-to-Roll printing manufacturing process, as shown in Fig. 3 depicting a Roll-to-Roll printing technique for large scale production of the biosensor stripes deposited onto flexible substrates, which leverages the benefits of printing technology, precise annealing procedures and in-line characterization techniques. The biosensor development, in large scale using a Roll-to-Roll system, process begins by employing printing techniques to deposit successive layers of biocompatible materials onto a continuous flexible substrate roll. These functionalities encompass conductive electrodes, insulating layers, and sensing elements, all of which play a pivotal role in the overall biosensor performance.

Figure 3a depicts a schematic representation of Roll-to-Roll printing technique for large scale production of the biosensor stripes deposited onto flexible substrates. Printing techniques that may be established in a Roll-to-Roll system consisting of screen printing notably, as well as Inkjet, slot die, laser printing and other. The Roll-to-Roll machine is elaborated by cutting-edge in-line and ex-situ characterization tools to provide detailed information about the quality of each printed layer including the biofunctionalized ones, for biosensor quality control 116, 117. Rolls 118 of the Roll-to-Roll system that drive the substrate and biosensor layers for printing and characterization, are also represented.

Figure 3b depicts a schematic representation of laser ablation 119 as a key step H in the Roll-to-Roll process to precisely pattern the conductive electrode layers 102 onto the flexible substrate 101. The method using laser ablation in the Roll-to-Roll process to precisely pattern the electrodes or any other biosensor components allows high-precision scribing of micro- to nano-scale features, minimizes heat-affected zones for material integrity, and enables real-time beam adjustments to ensure uniformity and reduce production defects.

The biosensor's stack structure is delineated as PET/Ag/REANE/CE/Dielectric/ Biofunctionalization, adhering to a well-defined configuration. In an analogous manner to laboratory-based production, all layers, inclusive of the metal electrode, are meticulously rendered via screen printing methodology or other, executed under ambient conditions.

The printing procedure entails consecutive printings of distinct materials, meticulously orchestrated in a standardized order. These layers, sequentially printed, encompass Contacts, Reference Electrodes, Working and Counter Electrodes, and the Dielectric Layer. The control of temperature and humidity conditions ensure the precise realization of these functional layers. Subsequent to the printing phase, thermal annealing is systematically applied to each functional layer. The critical insulation between the electrodes is accomplished utilizing a range of polymer-based dielectric pastes, chosen for their distinct attributes including chemical durability, resilience during annealing, and hydrophobic nature.

The biosensor's flexible substrate further amplifies its versatility, enabling comfortable and convenient usage in diverse clinical scenarios. The flexibility factor enhances patient comfort and facilitates usage across different anatomical positions.

To maintain stringent quality control, in-line characterization tools are seamlessly integrated into the manufacturing line (Fig. 3a). Eddy current testing is employed to monitor the electrical properties and identify defects in the deposited layers. Raman spectroscopy, on the other hand, is utilized to assess molecular composition and crystallinity, providing insights into the material's structural integrity. The Raman Spectroscopy is used to analyze the biofunctionalization layers as well as the conductive layers of the biosensor. A Raman Spectroscopy analysis of all layers of the biosensor is shown and of the biofunctionalization layers 106 with different concentration in Fig. 5b.

The crucial biofunctionalization step, which imparts biosensors with their specific recognition capabilities, is achieved through a micro dot deposition system. This system enables controlled and precise attachment of biomolecules onto the biosensor's working electrode surface, enhancing selectivity and sensitivity. The biofunctionalized biosensors are then subjected to further in-line characterization to validate their performance using eddy current and Raman spectroscopy. A selected number of these biofunctionalized biosensors are subjected to square wave voltammetry analysis. The obtained data is then utilized to create a reference curve that serves as a benchmark for biosensor performance evaluation. This innovative approach underscores the method's efficacy in producing biosensors with enhanced selectivity and sensitivity.

The measurements realized for the presentation and the use of the proposed technique are set out hereafter. In this remarkable approach, Atomic Force Microscopy (AFM) and Dynamic Light Scattering (DLS) are synergistically employed for comprehensive gold nanoparticle characterization, particularly in the context of biofunctionalization stages. AFM facilitates high-resolution imaging of individual gold nanoparticles, enabling precise visualization of their topographical features and size distribution. Simultaneously, DLS provides dynamic sizing information by analyzing the scattering pattern of laser light interacting with nanoparticles in solution, yielding hydrodynamic diameters. This dual-method strategy is instrumental in tracking the evolution of nanoparticle size across various biofunctionalization stages. From the initial pristine state, through ligand attachment, biomolecule conjugation, and functional layer deposition, AFM-DLS tandem analysis allows for direct comparison of nanoparticle size changes throughout each modification step. This innovative characterization approach holds promise for advancing tailored nanoparticle design in biomedical applications, nanomedicine, and targeted drug delivery systems, where accurately assessing and controlling nanoparticle size at each biofunctionalization stage is crucial for optimal performance and efficacy, Figs. 4a and 4b.

The present invention encompasses a method utilizing UV-Vis spectroscopy for nanoparticle characterization. Specifically, the technique is employed to measure the size of nanoparticles within a range of 30 - 60 nm by analyzing shifts in absorption peaks due to quantum size effects (Fig. 4c). The size of nanoparticles NPs should vary from 2 nm or less to 1800 nm or less, depending on the application wherein the shape can be but not limited to spherical, discoid, triangular, square, rectangular, or polygonal, cuboid, pyramidal, or hemispheric, nanotube, a nanowire, nanosphere, and other.

Additionally, the invention capitalizes on the distinct optical feature known as localized surface plasmon resonance (LSPR) exhibited by gold nanoparticles. Through UV-Vis spectroscopy, the strong absorbance band in the visible region (500 nm - 600 nm) resulting from LSPR is monitored to analyze the interactions of gold nanoparticles with surrounding media and to finely tune the optical properties of the nanoparticles. This novel application of UV-Vis spectroscopy offers precise insights into nanoparticle size and behavior, making it an invaluable tool in various fields including nanotechnology, materials science, and biomedicine.

The present invention pertains to an innovative method for the manufacturing of troponin biosensors, capitalizing on the potential of Raman spectroscopy to revolutionize the fabrication process. Raman spectroscopy is harnessed to provide comprehensive insights into material composition, aiding in optimizing biosensor quality and performance. In particular, the invention exploits the distinctive Raman scattering patterns exhibited by various materials employed in the biosensor assembly, enabling meticulous assessment during critical stages. Biomolecules and biofunctionalization steps are identified through high Raman shift values of peaks, enabling precise monitoring of surface modification processes crucial for achieving biosensor specificity. Within the lower 500 Raman shift range, intricate details of carbon molecules and the printing nuances of conductive electrodes are revealed, facilitating real-time evaluation of electrode integrity and uniformity. This inventive integration of Raman spectroscopy into troponin biosensor manufacturing transforms the field, offering a deeper comprehension of material properties, structure, and chemical processes, ultimately leading to the development of high-performance biosensors with significant implications for advanced cardiac diagnostics (Figs. 5a and 5b).

Fine tuning of the properties of the biosensor layers contributes to the rapid biosensor response time below 5 min and approximately 2 min. Fig. 6 shows the time response of the biosensor in samples of human blood and serum indicating the rapid measurement.

For each sensor, cyclic voltammetry (CV) was measured first followed by square wave voltammetry (SWV) as presented in Figs. 7a and 7b, respectively. For the voltammetry measurements using drop casting, 40 µl of Phosphate-buffered saline (PBS) were deposited on the WE, while 5 cycles were performed for each measurement in cyclic voltammetry.

In order to ensure accurate measurements of biofunctionalized biosensors, the range of voltage used during voltammetry analysis was reduced to [-0,2 V, -0,4 V]. This was due to the observation that high voltage may result in the destruction of the antibodies, causing instability in the biosensor measurements. It is noteworthy that for the analysis of empty biosensor platforms, a wider voltage range of [-0,8 V, 0,4 V] was used in the voltammetry system (CV, SWV). However, to ensure the preservation of the biofunctionalized biosensors, the voltage range was intentionally narrowed to [-0,2 V, -0,4 V]. This was deemed a necessary step to improve the accuracy and reliability of the measurements.

The present invention embodies a method of significant import, employing cyclic and square voltammetry to characterize troponin biosensors with exceptional efficacy. This method offers profound insights into the biosensors' functional process and performance metrics. By subjecting the troponin biosensors to cyclic and square voltametric analyses, an intricate understanding of their electrochemical dynamics is attained, elucidating the binding and release processes concerning troponin-a pivotal cardiac health biomarker. Cyclic voltammetry, in particular, unveils the redox phenomena intrinsic to troponin interaction, facilitating an in-depth exploration of surface-level interactions. Concurrently, square voltammetry provides a mechanism to discern concentration-dependent responses, permitting the generation of comprehensive reference calibration curves via troponin solutions of varied concentrations. This calibrated correlation between observed electrochemical responses and troponin concentrations guarantees the accurate quantification of troponin levels. The ingenious amalgamation of cyclic and square voltammetry underscores the potential of troponin biosensors as a potential diagnostic tool for swift, precise, and specific cardiac health assessment. This innovation carries transformative prospects for point-of-care diagnostics and personalized medical care, positioning troponin biosensors at the forefront of cutting-edge healthcare technologies.

Fig. 8 illustrates the measurements of the sensitivity versus potential of the cardiac troponin biosensor that a) detects the cTnT antigen in serum at different concentrations, b) in a drop of whole human blood. It can be noted that the cardiac biosensor has high sensitivity for troponin antigen with detection range from 0,01 ng/mL to 10 ng/mL.

A yet further example of the embodiment is its capabilities as screening, monitoring tool for life-threating conditions like cardiovascular disease (CVD). It can provide significant information about the quality of blood, blood vulnerability for thrombosis, heart attack, stroke or heart failure, lung embolism and other life-threating conditions, atherosclerosis. Fig. 9 depicts the troponin biosensor measurements in blood samples of human volunteers. The lowest the peak of current intensity or broader the peak area indicates the higher risk of CVD. Thus, it can be a convenient and useful tool for cardiologists and other physicians wishing to assess cardiovascular risk, plan and evaluate tailored risk factor intervention in high CVD risk subjects.

The biosensor is easy to use, portable, small in size and can give quantitative results of troponin elevation accurately, fast (<5 min) response time, from only a drop of blood from patients that undergo chest pain due to heart attack. it allows self-testing without the need of a clinician and can be easily adapted to wearables and point-of care systems.

A yet further example of the invention is an integrated biosensing device that translates raw biosensor signals into meaningful data that can be transferred to a data center, hospital, ambulance, or doctor's office for decision-making. Such translation can be complex, and variations in sensor signal intensities can pose challenges. This invention addresses these concerns through an integrated device that optimizes signal capture, conversion, analysis, and visualization.

The innovation highlights of the embodiment lie on the following: i) covalent bonding between bio-receptors and transducers, ensuring stable and efficient signal transmission, ii) Analog signals generated by biosensors are seamlessly converted to digital form, minimizing signal degradation and maximizing data accuracy, iii) device collects raw sensor signals in real time and employs advanced algorithms to filter out noise and artifacts, enhancing the quality of collected data, iv) the device scales sensor signals to a consistent baseline, ensuring that comparative measurements remain reliable, v) integrated software processes data in real time, providing instant insights into analyte presence and concentration levels, allowing for swift decision-making, vi) The integrated software processes data in real time, providing instant insights into analyte presence and concentration levels, allowing for swift decision-making.

The device displays results on an integrated panel or interfaces with a computer for comprehensive data visualization, aiding in result interpretation and reporting.

The remarkable biosensor presented herein introduces a transformative paradigm for troponin detection and heart problem diagnosis, monitoring and screening, The combination of high sensitivity, rapid response, integrated characterization tools, roll-to-roll manufacturing, and innovative biofunctionalization collectively positions this biosensor as an unparalleled solution in the field of cardiac diagnostics. Such sensor circumvents the need for enzymatic detection or fluorescence tagging, and enable compact, simple, inexpensive, point-of-care diagnostics.

Especially, it bridges the gap between the rapid cardiac tests and the point-of-care cardiac systems that lack data transferring.

A yet further example of this invention is its application for detecting the presence and quantify the levels of one or more biomolecular targets in biological samples or analytes including, but not limited to blood, serum, plasma, urines, tears, saliva, gastrointestinal secretions, cerebrospinal fluid, biopsy tissues or tumors, synovial fluid, feces, sputum, cyst fluid, amniotic fluid, peritoneal fluid, lung lavage fluid, semen, lymphatic fluid, extracellular fluid, cell culture media, cellular or nuclear extracts, viral, bacterial, cellular/tissue sample, water, air, soil sample, as well as a food industry, pharmaceutical, supplement cosmetics samples.

The Roll-to-Roll printing method allows the mass production of biosensors. The use of a roll-to-roll machine for biosensor fabrication represents a significant advancement over traditional methods. This scalable approach not only boosts production efficiency but also enables cost-effective manufacturing, making the biosensor solution accessible to a wider range of healthcare settings.

The method can be generally applied, notably in the case of biosensors and sensors for detection, screening and monitoring of diseases and health state, life-threating conditions like heart attack, stroke, aortic dissection, lung embolism, infections with pathogens, allergens, air pollutants, airborne microorganisms and nanosized viruses (e.g. influenza, HIV, SARs, SARs-CoV-2), inflammation and oxidative stress, cancer, diabetes, neurogenerative diseases, bacteria and microorganisms, wellbeing conditions, biomedical devices, sports, food processing and packaging, agriculture, pharmaceuticals, fine chemicals, flavor, fragrance, cosmetics, bioterrorism.

The applications of the device body of the present invention includes the following ones: wearables, biomedical, personalized medicine, point-of-care diagnostics, diagnosis, risk assessment and prognosis of diseases, sports, athlete safety monitoring, injury management, performance and fitness optimization, remote vital signs, health or recovery monitoring, personalized patient monitoring, food processing applications, agriculture, pharmaceuticals, research and drug development, bioterrorism, fine chemicals, flavor, fragrance, cosmetics, implantable devices, biomedical devices, medical equipment.

### References

*1.* Bambang Kuswandiz, and Ali A. Ensa, Paper-Based Biosensors: Trending Topic in Clinical Diagnostics Developments and Commercialization, J. of Electrochemical Society, 2020; 967 037509
*2.* Soohyun Kim & Jong-Hwan Lee. Current Advances in Paper-Based Biosensor Technologies for Rapid CO VID-19 Diagnosis. BioChip Journal 2022; 16:376-396
*3. Vanessa N Ataide, Lauro A Pradela-Filho, Wilson A Ameku, Masoud Negahdary, Thawan G Oliveira, Berlane G Santos, Thiago R L C Paixão, Lúcio Angnes, Paper-based electrochemical biosensors for the diagnosis of viral diseases. Review Mikrochim Acta 2023 Jun 27; 990(7):276*
4. G. Konoplev, D.Agafonova, L. Bakhchova, N. Mukhin, M. Kurachkina, MP. Schmidt, N. Verlov, A. Sidorov, A.Oseev, O. Stepanova, A. Kozyrev, A. Dmitriev, S. Hirsch. Label-Free Physical Techniques and Methodologies for Proteins Detection in Microfluidic Biosensor Structures. Biomedicines. 2022 Jan 18;10(2):207
*5.* A. Munawar, YOng, R.Schirhagl, MA Tahir, WS Khan, SZ Bajwa. Nanosensors for diagnosis with optical, electric and mechanical transducers. RSC Adv. 2019 Feb 27;9(12):6793-6803
*6.* J. Kneipp., Interrogating Cells, Tissues, and Live Animals with New Generations of Surface-Enhanced Raman Scattering Probes and Labels. ACS Nano2097, 11(2*)*
*7.* Raouia Attaallah, Amina Antonacci, Fabiana Arduini, Aziz Amine, and Viviana Scognamiglio. Nanobiosensors for Bioclinical Applications: Pros and Cons. (2020). In book: Green Nanoparticles (pp.117-149*)*
*8*. Luciano Babuin and Allan S. Jaffe. Troponin: the biomarker of choice for the detection of cardiac injury. CMAJ.2005 Nov 8; 173(10): 1191-1202
*9.* Xi-Ying Wang, Fen Zhang, Chi Zhang, Liang-Rong Zheng and Jian Yang. The Biomarkers for Acute Myocardial Infarction and Heart Failure. Biomed Res Int. 2020; 2020: 2098035
*10.* Yader Sandoval, Fred S. Apple, Simon A. Mahler, Richard Body, Paul O. Collinson, Allan S. Jaffe. High-Sensitivity Cardiac Troponin and the 2021 AHA/ACC/ASE/CHEST/SAEM/SCCT/SCMR Guidelines for the Evaluation and Diagnosis of Acute Chest Pain. Circulation. 2022;146:569-581*.*
*11.* Connie W. Tsao, Aaron W. Aday, Zaid I. Almanzooq, Alvaro Alonso, Andrea Z. Beacon, Marcio S. Bittencourt, et al. Heart Disease and Stroke Statistics-2022 Update: A Report From the American Heart Association. Circulation. 2022;145:e153-e639*.*

## Claims

1. Biosensing device for diseases detection including for diagnoses, monitoring and screening thereof, esp. at nanoscale, comprising a nano-technology enabled biosensing means, which is biofunctionalized for detection of a selected set of biomarkers and for quantitative measurements thereof, and an electronic device (110) with internet connectivity for data transmission, **characterized in that** said biosensing device (100) comprises a Flexible Substrate means (101) as well as Contacts-Electrodes means (102), a Reference Electrode (RE) means (103), Working & Counter Electrode (CE) means (104), a Dielectric means (105) and a Biofunctionalization means (106), which are arranged as a set of successive layers, wherein said electrode means (102) are arranged onto said flexible substrate means (101), and wherein said biofunctionalization means (106) detects said biomarkers and monitors same.

2. Biosensing device according to claim 1, **characterized in that** said biosensing device (100) is arranged as a stack structure consisting of a superposition of said layers comprising said Flexible Substrate layer (101) supporting said Contacts-Electrodes layer (102), Reference Electrode (RE) layer (103), Working & Counter Electrode (CE) layer (104), Dielectric layer (105) and Biofunctionalization layer (106) esp. in this order, which are formed with a mutually similar peripheral shape, in particular with a substantially square or rectangular like outline, wherein the pattern of each layer ultimately defines the shape of the biosensing device, more particularly wherein said layers (102,...,106) are made of mutually biocompatible materials.

3. Biosensing device according to claim 2, **characterized in that** it further comprises an insulation means consisting of an insulation layer, which is provided between said electrodes means, particularly which is tailored to said similar peripheral shape with said similar outline, which is made of at least one polymer-based dielectric paste, which is applied to cover said biosensing device,
wherein said insulation means resp, insulation layer mutually isolates said electrodes means encompassing said working electrode & counter electrode layer (104), and reference electrode layer (103), and maintains an electrical isolation between said electrodes means, thereby preventing electrical contact between said electrodes means and avoiding potential merging of their functions, which allows said working electrode (WE) and counter electrode (CE) layer (104), and reference electrode (RE) layer (103) to operate independently, esp. contributing to preserve the integrity of electrochemical signals generated by the interaction between said analyte or biomarkers and the biosensing device (100), thus enabling an accurate detection and quantification of said biomarkers.

4. Biosensing device according to one of the preceding claims 1 to 3, **characterized in that** said biosensing device (100) comprises a biosensor stripe and its components involving Electrodes (102) onto flexible substrate (101), Reference Electrode (RE) (103), Working and Counter Electrode (CE) (104), Dielectric (105), and Biofunctionalization layer (106),
wherein said biosensing device (100) produces a biochemical signal and the biosensing electronic device (110) is provided with reading means for reading said biochemical signal, particularly wherein said biosensing electronic device (110) has an external surface and its components notably consisting of a Monitor display (115),
as well as an internal surface and parts composed of a Connector for Flexible Flat Cables (FFC)/Flexible Printed Circuits (FPC) (111), a Printed Circuit Board (PCB) (112), Electronic Components (113), Electronic Connection Parts (114) Micro-USB or Type-C, Bluetooth module, Wi-Fi module.

5. Biosensing device according to one of the preceding claims 1 to 4, **characterized in that** said biofunctionalization means (106) comprises nanoparticles, and/or nanotubes, diagnostic agents, antibodies (Ab) or mixtures thereof, wherein said nanoparticles (NPs) are synthesized as a diversity of conductive nanoparticles (NPs) notably inorganic ones, esp. gold, silver, aluminum, platinum, palladium, graphite, silver or copper, and/or organic ones, esp. conductive polymeric nanoparticles (NPs) made of glassy carbon, conductive materials, thereby enhancing the selectivity and sensitivity of the biosensing device.

6. Biosensing device according to claim 5, **characterized in that** said nanoparticles have a nanoplasmonic structure, resp. nanotube, nanowire, nanosphere structure, in particular having dimensions ranging from ≤ 2 nm, ≤ 1800 nm respectively,
wherein said nanoparticles (NPs) are functionalized by means of at least one diagnostic agent, particularly wherein said at least one diagnostic agent consists of an antibody or antibody fragment or nanobodies thereof, which provide the recognition of the said specific biomarker of interest, more particularly wherein said at least one diagnostic agent comprises a first capture agent and a second capture agent, wherein said first agent is specific for said second capture agent, and the said second capture agent is specific for at least one biomarker and biomolecular targets.

7. Biosensing device according to one of the preceding claims 1 to 6, **characterized in that** said Reference Electrode (RE) means (103) are made of Silver/Silver Chloride (Ag/AgCI) paste, particularly with a sheet resistance of less than 100 Ω/sq.,
and for silver nanoparticles and silver nanowires, *more* particularly as well as Ag/AgCl nanoparticles, Graphene Oxide (GO) Ink mixed with Ag/AgCl nanoparticles, noble metals esp. gold or platinum as well as their metal oxide nanoparticles, metal oxide inks;
and/or **in that** said Working (WE) and Counter Electrodes (CE) means (104) consist of carbon inks like graphite, carbon or graphene oxide (GO) paste and their nanotubes, noble metals, esp. gold, as well as platinum, silver, copper and palladium and their nanoparticles, conductive polymers, metal oxide, particularly wherein said working and counter electrodes means (104) are comprised of conductive carbon sensor paste, each with a sheet resistance of less than 75 Ω/sq.

8. Biosensing device according to one of the preceding claims 1 to 7, **characterized in that** said contact electrodes means are made of silver (Ag) paste, and/or Graphene, in particular as well as Silver Nanowires, Conductive Polymers, Nanowires, Metal Nanoparticles esp. gold and silver ones, Metal Oxides, Carbon-based inks, Flexible Metal Film esp. copper or aluminum.

9. Biosensing device according to one of the preceding claims 1 to 8, **characterized in that** said Dielectric means (105) comprise a diversity of inks, polymers notably polyimides, polyethylene, polypropylene, aluminum oxide (Al₂O₃) and zinc oxide (ZnO), and/or
wherein said Dielectric means (105) are made of ceramic materials notably with high dielectric constants, dielectric powders mixed with binders, crosslinkers, solvents and curing agents, glass-ceramic powders mixed with binders, polymer-ceramic composites, silicone-based dielectrics, dielectric elastomers, ceramic-polymer hybrid materials;
and/or wherein said flexible substrate (101) is made of a material selected among Polyimide (PI), in particular also Polyethylene Naphthalate (PEN), as well as Flexible Glass, Paper-Based Substrates, woven and non-woven fabrics, Polymer Blends, possibly Polyester (PET).

10. Method for manufacturing a biosensing device as defined in one of the preceding device claims 1 to 9, **characterized in that** said biosensing device is fabricated by means of a roll-to-roll system device (130), wherein the process is started with a printing step (A) to deposit successive layers (102,...,106) of biocompatible materials onto a continuous flexible substrate (101) roll (118), esp. by screen printing, inkjet or flexography printing, esp.
wherein the printing process consists of consecutive printings of different materials in a standard order, wherein these layers, in printing order, are said Contacts, Reference Electrodes (103), Working and Counter Electrodes (104) and Dielectric Layer (105),
wherein Thermal annealing is applied to each functional layer after the printing process, particularly wherein an array of cutting-edge step (B) in-line and an ex-situ characterization step (C) are incorporated in the biosensing device manufacturing process, thereby comprising ellipsometry spectroscopy, Raman spectroscopy, wherein the quality and functionality of each production step is monitored synergistically in a monitoring step (D).

11. Method for manufacturing a biosensing device according to claim 10, **characterized in that** the method further comprises a laser ablation step (H) in the Roll-to-Roll process to precisely pattern the conductive electrode layers (102) onto the flexible substrate (101), wherein the laser ablation process allows high-precision scribing of micro- to nano-scale features, further minimizes heat-affected zones for material integrity, and enables real-time beam adjustments to ensure uniformity and reduce production defects.

12. Method for mass production of a nanotechnology enabled biosensing device according to claim 10 or 11, **characterized by** a high selectivity and specificity for early detection, screening and prevention of life-threating conditions esp. heart attack and its internet connectivity for data transferring and streamlining patient care,
wherein a biofunctionalization step (F) is conducted after the electrodeposition step (E), wherein it involves the deposition of several bio-layers on the working electrode (104), starting with an adhesive layer (161) which acts as a transport layer for the charge carriers, thereby further enhancing the biosensing device's performance, particularly wherein said adhesive layer (161) is an ionic liquid crystal, esp. Potassium ferrocyanide (K4[Fe(CN)6]), methylene blue, thionine and quinones,
wherein the next layer (162) is a homogenous compound made of nanoparticles (NPs), esp. gold ones and Antibodies (Ab), which detect the addition of antigen on the working electrode (104) surface,
wherein finally, a layer of blocking agent (163) is added through an addition step (G), esp. Glycine, Mercaptoundecanol (MCU), Polyethylene glycol (PEG), Bovine serum albumin (BSA), to enhance the biosensing device's sensitivity and decrease false signals in case.

13. Method according to claim 12, **characterized in that** a diversity of nanoparticles (NPs) is used notably inorganic ones esp. gold, as well as silver, magnetic nanoparticles (NPs), esp. iron oxide ones, semiconductor quantum, carbon nanotubes, liposomes, metal oxide nanoparticles (NPs), esp. zinc oxide or titanium dioxide, polymeric nanoparticles (NPs), silica ones,
and/or wherein said nanoparticles are functionalized with a diagnostic agent esp. an antibody (Ab) or antibody fragment thereof, a receptor, a recombinant fusion protein, a peptide or a nucleic acid molecule that provide the recognition of the targeted specific biomarker.

14. Method for production of a nanotechnology enabled biosensing device according to one of the claims 1 to 9, of a cardiac biosensing device, **characterized in that** a cardiac biomarker in patients, esp. consisting of troponin levels, is detected including from only a small quantity of blood, even a drop, and wherein the qualitative and quantitative measurements of the said cardiac biomarker, is transmitted to the nearest therapeutic intervention agent for a therapeutic intervention, particularly also including cardiovascular biomarkers.

15. Method according to one of the claims 10 to 14, **characterized by** the biofunctionalization of said biosensing device yielding the detection of ultra-low levels of the targeted biomarkers of interest for primary therapeutic intervention, particularly wherein this biosensing device is applied to wearables, as well as medical services notably including biomedical, personalized medicine, pharmaceuticals.
